# EUROPEAN PATENT SPECIFICATION

(11) **EP 2 838 463 B1**
(45) Date of publication and mention of the grant of the patent: **08.11.2017**
(21) Application number: 13778281.9
(22) Date of filing: 16.04.2013
(51) Int. Cl.: A61B 5/00, A61B 5/06, A61M 5/00, A61B 34/37, A61B 34/20, A61B 34/30, A61B 90/00

(54) **DUAL-MODE STEREO IMAGING SYSTEM FOR TRACKING AND CONTROL IN SURGICAL AND INTERVENTIONAL PROCEDURES**
DOPPELMODUS-STEREOBILDGEBUNGSSYSTEM ZUR VERFOLGUNG UND STEUERUNG IN CHIRURGISCHEN EINGRIFFSVERFAHREN
SYSTÈME BIMODE D'IMAGERIE STÉRÉO POUR SUIVI ET COMMANDE DANS DES INTERVENTIONS CHIRURGICALES ET DES TECHNIQUES INTERVENTIONNELLES

(30) Priority: 16.04.2012 US 201261624665 P
(43) Date of publication of application: 25.02.2015
(73) Proprietor: Children's National Medical Center, Washington, DC 20010 (US)
(72) Inventor: AZIZIAN, Mahdi, Sunnyvale, CA 94086 (US); KIM, Peter C.W., Washington, DC 20016 (US); KRIEGER, Axel, Alexandria, VA 22305 (US); LEONARD, Simon, Washington, DC 20002 (US); SHADEMAN, Azad, Washington, DC 20008 (US)
(74) Representative: Gill Jennings & Every LLP
(86) International application number: PCT/US2013/036773
(87) International publication number: WO 2013/158636

(56) References cited:
- WO-A2-03/077749
- WO-A2-2007/136768
- US-A- 5 928 137
- US-A1- 2003 192 557
- US-A1- 2004 106 916
- US-A1- 2005 182 321
- US-A1- 2007 273 610
- US-A1- 2008 312 561
- US-A1- 2009 248 038
- US-A1- 2009 268 010
- US-A1- 2011 082 369
- US-B2- 7 008 373
- US-B2- 8 090 194

## Description

### BACKGROUND OF THE INVENTION

### Field of the Invention

The present embodiments relate generally to apparatuses and methods for tracking and control in surgery and interventional medical procedures.

### Description of the Related Art

There is currently no technology for robust image-guidance in automated surgery. What is available in the market as so called "robotic surgery" is truly just robot-assisted surgery because the robot only follows direct commands of the surgeon with very little intelligence or autonomy. For instance, US 2009/268010 A1 describes such background art and discloses a robotic surgical system comprising an illumination channel, a stereoscopic optical channel and another optical channel which are held and positioned by the robotic surgical system. A first capture unit captures a stereoscopic visible image from the first light from the stereoscopic optical channel while a second capture unit captures a fluorescence image from the second light from the other optical channel. An intelligent image processing system receives the captured stereoscopic visible image and the captured fluorescence image and generates a stereoscopic pair of fluorescence images. An augmented stereoscopic display system outputs a real-time stereoscopic image comprising a three-dimensional presentation of a blend of the stereoscopic visible image and the stereoscopic pair of fluorescence images.

Some research groups have looked into closing the loop of control for surgical robots with existing sensors, however special conditions and considerations applied to operations in-vivo, make it extremely difficult to achieve such goals.

### SUMMARY OF THE INVENTION

The invention is defined in the appended claims. The present embodiments address at least this problem by introducing a robust tracking technique which requires minimal changes to the current robot-assisted surgical workflow and closing the loop with an effector function.

### BRIEF DESCRIPTION OF THE DRAWINGS

A more complete appreciation of the embodiments described herein, and many of the attendant advantages thereof will be readily obtained as the same becomes better understood by reference to the following detailed description when considered in connection with the accompanying drawings, wherein
Figure 1 shows the overall structure of the proposed embodiment of the disclosure in semi-autonomous mode where the surgical tasks are partially automated by visual servoing;
Figure 2 shows the embodiment of the system in the manual or master-slave robot-assisted mode;
Figure 3 represents an embodiment of the system with supervised autonomy;
Figure 4 shows a spectral range of the excitation and emission lights which clearly describes the distinct spectral ranges associated with the main components involved: i.e., hemoglobin's (oxygenated and deoxygenated), water and the fluorescent dye. Fluorescent dyes with different spectral ranges for excitation and emission can be synthesized (e.g. Cyanine dyes);
Figure 5 illustrates an example of markers placed around a phantom cut;
Figure 6 illustrates images captured using a near infrared camera with two example fluorescent agents;
Figure 7 illustrates stereo image formation and triangulation to extract three dimensional (3D) coordinates of NIR markers according to one embodiment;
Figure 8 illustrates a flow diagram for an exemplary robotic operation algorithm;
Figure 9 illustrates a flow diagram for another exemplary robotic operation algorithm;
Figure 10 illustrates a flow diagram for a method according to one embodiment; and
Figure 11 illustrates a block diagram of a computing device according to one embodiment.

### DETAILED DESCRIPTION OF THE PREFERRED EMBODIMENTS

Aspects of the invention are set out in the appended claims.

Some variants of embodiments of the technology are listed below:

The technology can be used with multiple dyes with excitation/emission at different wavelengths. This can be applied to have inherently different markers for tracking multiple objects. In one embodiment, fluorescent dyes A and B are used to mark the two sides of a tubular structure prior to automated anastomosis.

The markers can be applied to the targets both internally and externally. The fluorescent dye can be attached to the target by clips, staples, glue or can be applied by painting or spraying. The dye can also be injected to the tissue to mark specific points or can be injected through blood. The dye can be selected in order to bind with specific types of cells to mark specific structures (such as tumors).

Providing "no-fly zones" or "virtual fixtures" to prevent the surgical tools from approaching critical structures: In this embodiment, the surgeon marks the critical structures prior to the task and the marked borders will be tracked using the dual-mode imaging technology. The coordinates will be used to force constraints on the motion of the surgical tools during the automated or semi-automated task. It can also be used to provide alarms (visual/audio or haptic) in manual tasks.

The imaging system can be monoscopic and provide two-dimensional location of the tracked points which can potentially be used for image-based visual servoing. The imaging system can be stereoscopic and provide three-dimensional location of the tracked structures and therefore be used for image-based or position-based visual servoing.

The embodiments of the technology can be applied for automated or semi-automated applications. It can also provide guidance for manual operations through visual, audio or haptic feedback.

Automation of a surgical procedure is a very challenging task. The surgical scene is dynamically changing, deformable organs may occlude surgeon's view and variations in illumination make it extremely difficult to robustly track any target and object inside the patient's body. Several attempts have been made to develop image-based tracking algorithms for minimally invasive and/or open surgeries but depend on special conditions and are not robust; therefore cannot be used to control any of the surgical tools or to automate parts of a surgery.

The present embodiments address these limitations by using a dual-spectrum imaging device which can image in the visual spectrum as well as in near-infrared (NIR) spectrum. The surgeon places fluorescent markers on the locations which should be tracked (e.g., tools and tissue); The excitation light generated by the imaging device causes the fluorophores to emit NIR light which will be detected by the imaging device. As a result, the system has a high signal to noise ratio (SNR) because of (a) limited autofluorescence of the tissue compared to the fluorescent dyes, and (b) lack of other NIR sources in the patient's body. This high SNR makes any tracking algorithm more robust and reliable. NIR light has a good penetration in the tissue as opposed to the visible light; this makes it possible to track an object even if it is occluded by another organ, flipped over, covered by blood, etc. A combination of visual and NIR images can be used to make image-based tracking algorithms even more robust.

One embodiment describes a system for automation of surgical tasks. It is based on deploying fluorescent markers on the organ under surgery and/or on the surgical tool, tracking the markers in real-time and controlling the surgical tool via visually servoing.

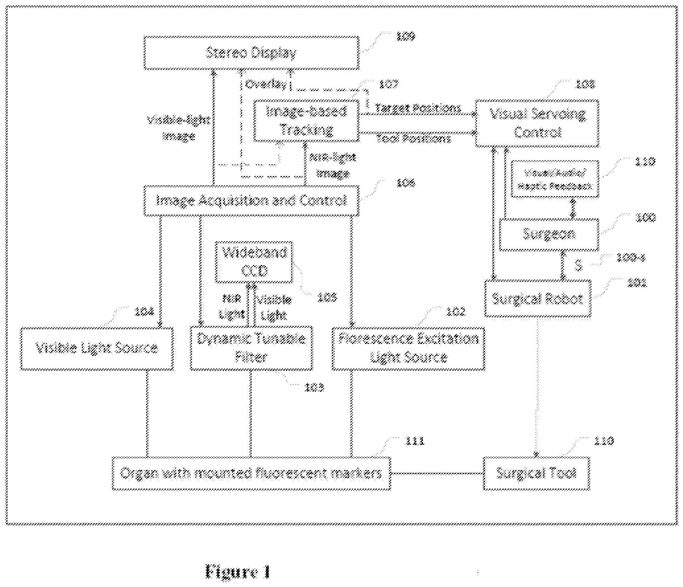

Figures 1, 2 and 3 represent different modes of the operation for the system. Fluorescent markers are deployed on the organ (e.g. two sides of a bile duct to be anastomosed) through spraying, painting, attachment, or other techniques 111. The markers can also be generated by techniques such as by mixing fluorescent dye, e.g. Indocyanine green (ICG), with a biocompatible glue e.g. Cyanoacrylate-ICG mix, delivered by pipette, or spray. The markers can also be generated by any element which provides sufficient fluorescence.

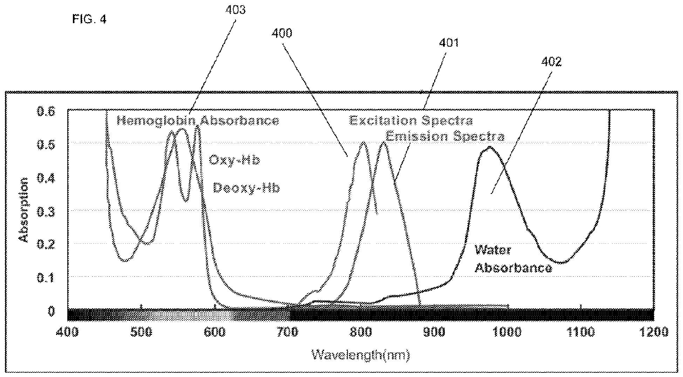

Figure 4 shows spectral characteristics of a fluorescent dye. The separation between excitation and emission wavelengths reduces interference caused by the excitation light source significantly. Fluorescent dye can be chosen to have its emitted wavelength beyond the visible light range in order to achieve a high signal to noise ratio in the near-infrared images. Also having the fluorescent emission 400 and excitation 401 wavelengths away from peak absorption wavelengths of water 402 and hemoglobin 403 provides a stronger signal and makes it easier to track fluorescent markers in presence of soft tissue (with high water content) and blood.

In one embodiment, multiple different markers are used to help track multiple structures, organs, and tools. Using different markers reduces the error rate for tracking, since the number of similar markers is reduced. Differentiation of markers can be achieved by having different size or volume and/or shape of the markers and or using dyes with excitation/emission at different wavelengths. In one embodiment, markers with 3 micro liters volume and markers with 6 micro liters volume are used to mark the two sides of a tubular structure respectively prior to automated anastomosis. In another embodiment, a fluorescent dye emitting at 790nm corresponds to the no-fly zone while a different wavelength 830nm corresponds to an edge of a structure.

In one embodiment, each structure (i.e. organ, stream segment) is assigned a structure identification number. Likewise, when the surgeon marks a structure at the anastomoses site, each marker is automatically assigned a unique identification number and is automatically labeled with the structure identification number to which it is attached. As the markers are tracked, the label of each marker is used to determine which structure it belongs and its overlay color. This tracking may be performed using tables or databases implemented by a computer processor and corresponding software instructions.

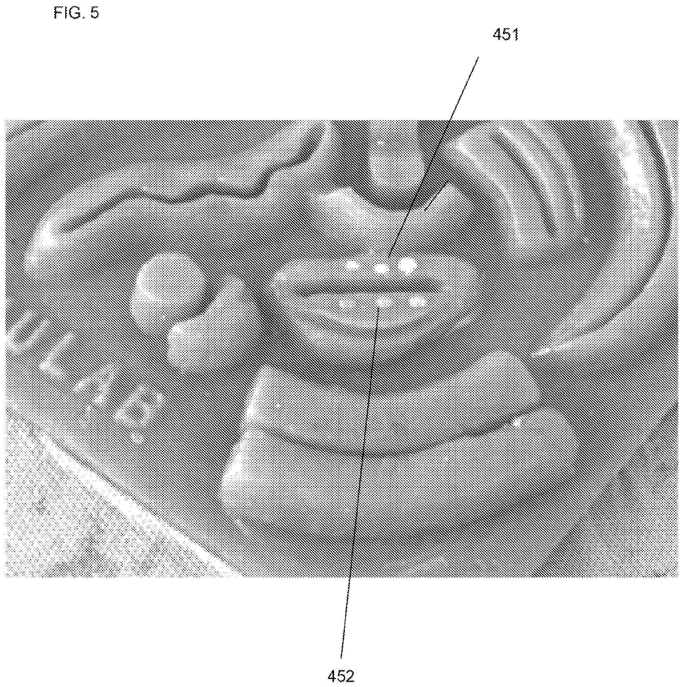

Figure 5 illustrates markers placed on around a phantom cut. A first set of markers 451 on the top side of the cut are labeled with a first color (e.g. yellow), and a second set of markers 452 on the bottom side of a cut are labeled with a second color (e.g. green).

Figures 1-3 illustrate two light sources 102 and 104 illuminate the scene. One light source 104 is a visual light source that makes it possible to acquire normal images of the organs. The other light source 102 is a narrow-band source of light (e.g. in the near infrared range) that is chosen according to the excitation wavelength of the fluorescent material. A "dynamic tunable filter" 103 changes the filter's characteristics in real-time to pass the visual light and the light emitted by the fluorescent material alternatively. At each moment the filter 103 only passes one type of light and suppresses the other. A wide-band CCD 105 captures images of the received light from either source. The light sources 102 and 104, the tunable filter 103 and the image capturing in the CCD 105 are controlled and synchronized by the image acquisition and control module 106. The image acquisition system runs at a high frame rate (e.g. 60Hz to 120Hz) and therefore it acts like two imaging systems with different wavelengths. In another embodiment, NIR and visual light is split by using either a beamsplitting or a dichromatic prism, with two CCDs capturing images, one for the visual spectrum and one for the NIR spectrum. In yet another embodiment, there are separate light paths for both NIR and visual light to two separate CCDs. All these concepts can be simply extended to a multiple wavelength imaging system. Image acquisition and control module 106 also captures and digitizes the images and provides them to two higher-level modules 107 and 109. The stereoscopic display 109 provides the acquired visual images; it can also display fluorescent images as a color coded overlay or display an augmented reality image by overlaying the target points detected by the image-based tracking module 107. The image-based tracking module 107 applies image processing algorithms to detect the fluorescent markers in order to track the tools and the organ. Visual features can also be used for tracking.

The image-based tracking module 107 also includes a tracking module that performs pre-processing of the NIR image and visual tracking based on the processed image information. In one embodiment, the pre-processing algorithm involves image processing algorithms, such as image smoothing, to mitigate the effect of sensor noise; image histogram equalization to enhance the pixel intensity values, and image segmentation based on pixel intensity values to extract templates for the NIR markers. The visual trackers are initialized first. The initialization of the visual trackers starts by detection and segmentation of the NIR marker. Segmentation is based on applying an adaptive intensity threshold on the enhanced NIR image to obtain a binary template for the NIR markers. A two dimensional (2D) median filter and additional morphology-based binary operators (binary image processing algorithms such as image erosion and dilation) may be applied on the binary template to remove segmentation noise. The binary template may be used as a starting base for visual tracking of NIR markers using visual tracking algorithms. After pre-processing and segmentation, the NIR template is a white blob on a darker background, which represents the rest of the surgical field in the NIR image.

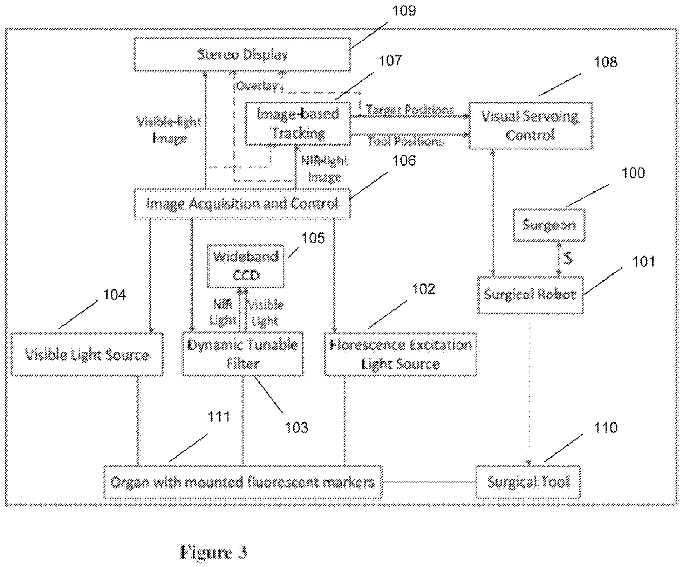

In Figures 1 and 3 representing "semi-autonomous" and "supervised autonomous" modes respectively, the surgeon 100 interacts with the surgical robot as a supervisor (100-s) taking over control through a master console whenever required. In the semi-autonomous mode (Figure 1) the surgeon 100 also provides commands to the visual servoing controller 108 during the operation. The visual servoing controller 108 receives the tracking information from the image-based tracking module 107, combines these with the intraoperative commands from the surgeon 100 and sends appropriate commands to the robot in real-time in order to control the surgical robot 101 and the surgical tool(s) 110 to obtain a predetermined goal (e.g. anastomosis). The surgeon 100 can be provided with visual, audio or haptic feedback 110 while he/she is looking at the stereoscopic display.

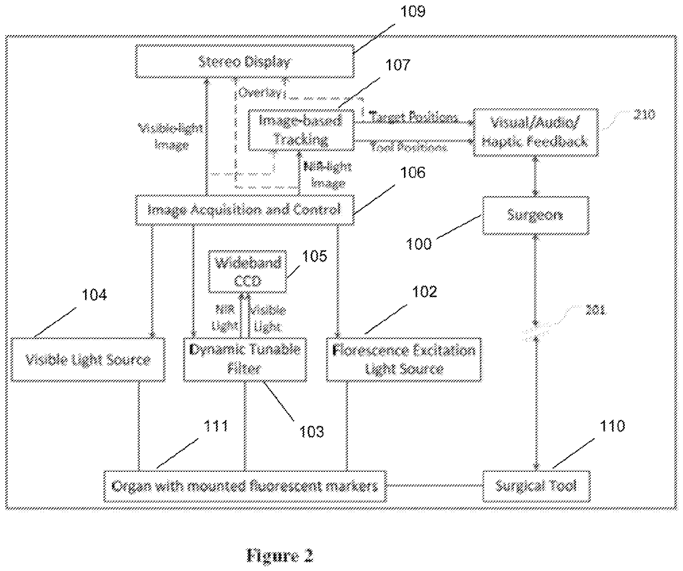

In manual mode (Figure 2), the surgeon controls the surgical tool manually (like in conventional laparoscopic surgery) or through master-slave control (201) of a robot arm. The surgeon receives visual feedback through the stereoscopic display (109) and may also be provided with other visual, audio or haptic feedback but the control loop is solely closed through the surgeon.

In autonomous mode (Figure 3), the control loop is solely closed via visual servoing except when the surgeon stops the autonomous control and takes over control (100-s) to prevent a complication, correct for a wrong action, or other reasons.

The tracked visual markers are used to guide the motion of the robot. Each visual marker is represented by a representative vector of numbers, which is typically called a visual feature. Examples of visual features are coordinates of the centers of NIR markers extracted from the binary image, and/or their higher-order image moments (such as their area in terms of number of pixels).

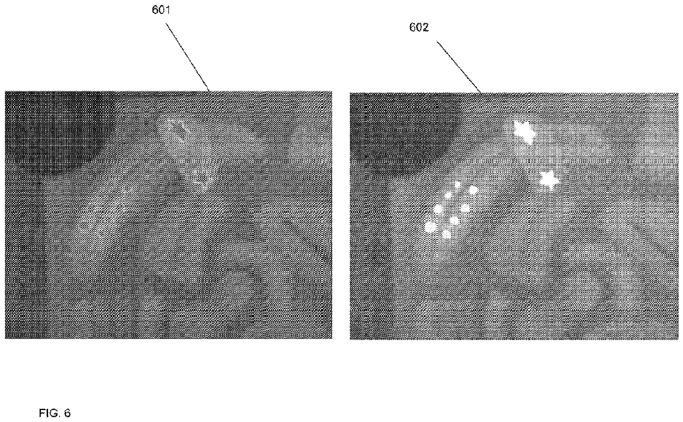

Figure 6 illustrates images captured using a NIR camera with two example fluorescent agents. Image 601 illustrates a binary image after image processing. Image 602 illustrates data that can be used as visual tracking information.

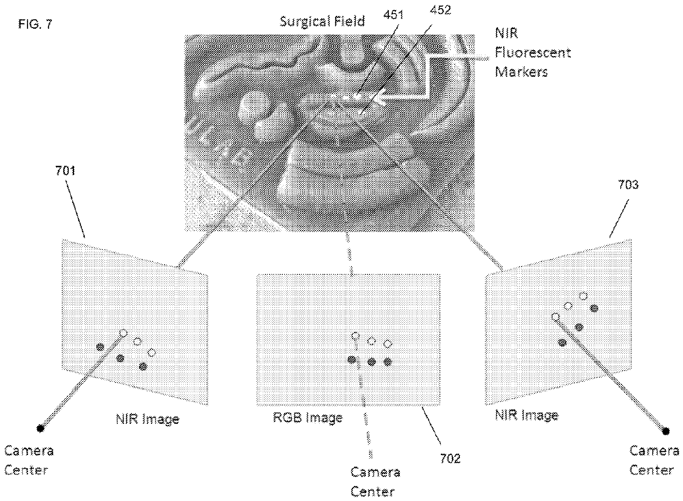

Robot motion is performed by transforming the sensor measurements into global Cartesian coordinate form for the robot. In one embodiment, the NIR and tool markers are tracked in the stereo images to compute the 3D coordinates of the marker or tool with respect to the surgical field, as shown in Figure 7.

In particular, Figure 7 illustrates stereo image formation and triangulation to extract three dimensional (3D) coordinates of the NIR Markers. These 3D coordinates are used by the robot motion control algorithm in open-loop or closed-loop architecture. The error between the tool position and the marker position is calculated and used to generate the desired tool displacement.

When the motion control feedback loop is closed in the sensor space, the effect of calibration errors is limited. This is desired for supervised autonomy. Vision-based, closed loop feedback, motion control of robots is called visual servoing. There are two main approaches to visual servoing based on control architecture: position-based visual servoing (PBVS) and image-based visual servoing (IBVS). Both approaches are viable options. In PBVS, the position of the robotic tool is estimated and the error is estimated based on the estimated position and the goal tool position. In IBVS, the image features are used directly to compute the task error in the image space, such that when the robotic tool is at the goal position the task error is zero. Both control approaches generate motions that drive the error to zero.

The NIR based robot motion control is a core technology which has not been developed in the past. Previous methods and apparatuses for NIR based imaging (without robot control, Frangioni 2012, US 8,229,548 B2) and NIR based display (Mohr and Mohr, US 2011/0082369) fail to consider robot motion control or any control whatsoever. With a stereo imaging system consisting of two NIR cameras with appropriate filters, a properly excited NIR agent can be seen in both stereo images. Image processing and visual tracking algorithms, such as the algorithms described above as being implemented by the image-based tracking module 107, are utilized to visually track each NIR marker in the image. The 3D estimate of a marker position is found by triangulation of the NIR marker image as seen in both left 701 and right 703 NIR stereo image pairs. The 3D estimate of the NIR marker can then be re-projected as an overlay in the RGB image 702. The tool position is also found from the stereo image pair. The stereo NIR system can be replaced by a 3D sensing camera capable of NIR observation.

The embodiments described herein are also very useful in non-stereo applications. For example, the system can be implemented for mono camera applications. For manual and master-slave modes (Figure 2), mono camera images are sufficient. In semi-autonomous mode, depth of the target points is important for the robot to perform positioning tasks. Stereo imaging can provide depth information. However, there are other depth sensors available that do not require a second camera, such as time of flight, conoscope, laser, and other depth cameras. This disclosure would also work with single cameras for manual and master-slave mode. For semi-autonomous mode, the present embodiments would also work with single camera and an additional depth sensor.

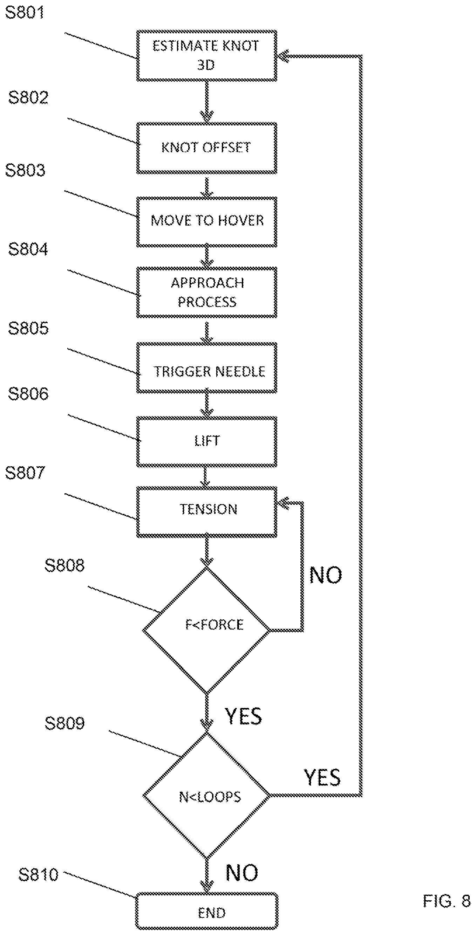

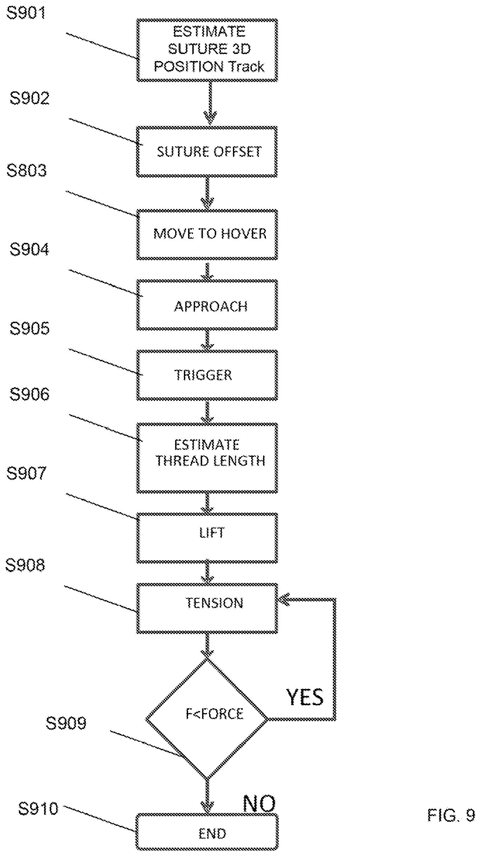

Figures 8 and 9 illustrate two flow charts of exemplary robotic operation algorithms implemented by the system. For instance, Figure 8 illustrates an algorithm for robotic knot tying and Figure 9 illustrates an algorithm for robotic suturing. The marker positions are used to estimate knot 3D position (Figure 8) and suture 3D position (Figure 9). The flow charts describe the robotic motions that follow position estimation.

As is shown in Figure 8, the robotic operation algorithm begins in step S801 with the execution of an estimation of the knot. In step S802, the knot offset is determined and communicated to the robot. In step S803, the robot moves to hover above the suture placement. In step S804, the approach process is performed. In the approach process, the robot takes into account the position information obtained based on the detected markers. Thus, the robot uses visual servoing to guide the needle toward the NIR marker. In step, S805 the needle is triggered. This trigger could be met when the robot has come within a predetermined distance of the knot. In step S806, the robots lifts the tool to pull enough thread. In step S807, the robot lifts the tool furthermore until a sufficient tension F is measured in the thread. This process is repeated for the number of desired loops in the knot.

Figure 9 is an example of a robotic suturing process. In step S901, the suture 3D position track is estimated. In step S902, the suture offset is determined. In step S903, the robot moves to hover above the suture placement. In step S904, the robot uses visual servoing to drive the needle toward the placement indicated by the NIR marker. In step S905, the suture is triggered. In step S906, an estimation of the length of thread is calculated. Using this estimation, in step S907, the robot lifts the needle to complete the suture. In steps S908, S909, robot lifts the needle until a tension of F is measured in the thread. The system exits if the tension is greater than F.

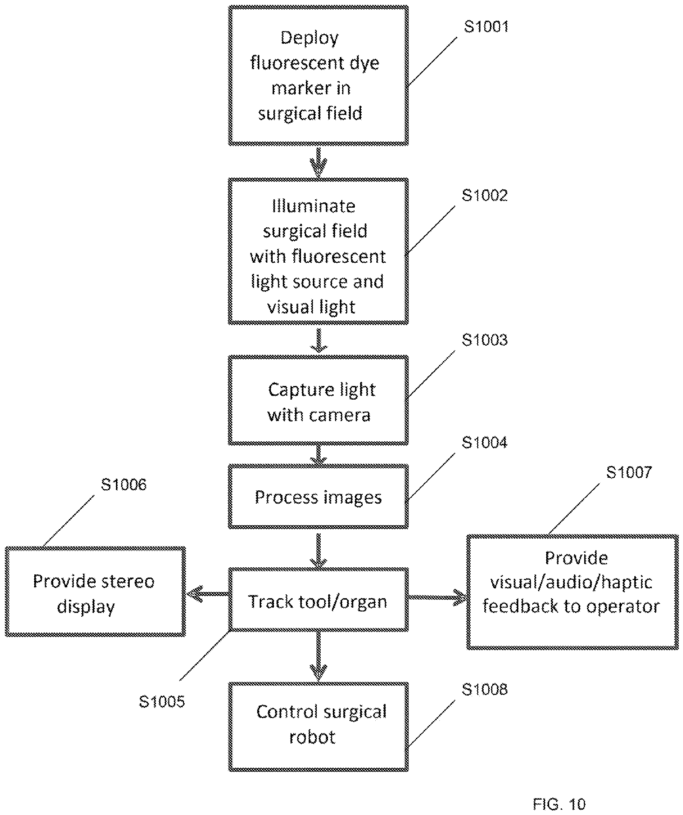

Figure 10 illustrates an overall process according to one embodiment. In step S1001, fluorescent dye markers are deployed to a surgical field. The dye markers can be deployed, for example, by spraying, painting, attachment, tissue injection, intravenous injection etc. In step S1002, the surgical field is illuminated with fluorescent and visible light sources. In step S1003, light is captured with a camera. The light captured by the camera is both in the visible and IR range. In step S1004, the resulting images are processed by the image processing algorithms described previously in order to identify markers in the image. In step S1005, based on the detected markers, the tool or organ, which is marked by the markers is tracked. This tracking is described in detail previously and includes determining the location of tools, organs, or other marked portions of the subject within the surgical field based on markers which are associated with respective elements. In step S1006, a stereo display is provided based on the tracking. In step S1008, visual, audio and haptic feedback is provided to the surgeon. In step S1009, a robot is controlled based on the tracking.

Certain portions or all of the disclosed processing, such as the image processing and visual tracking algorithms, for example, can be implemented using some form of computer microprocessor. As one of ordinary skill in the art would recognize, the computer processor can be implemented as discrete logic gates, as an Application Specific Integrated Circuit (ASIC), a Field Programmable Gate Array (FPGA) or other Complex Programmable Logic Device (CPLD). An FPGA or CPLD implementation may be coded in VHDL, Verilog or any other hardware description language and the code may be stored in an electronic memory directly within the FPGA or CPLD, or as a separate electronic memory. Further, the electronic memory may be non-volatile, such as ROM, EPROM, EEPROM or FLASH memory. The electronic memory may also be volatile, such as static or dynamic RAM, and a processor, such as a microcontroller or microprocessor, may be provided to manage the electronic memory as well as the interaction between the FPGA or CPLD and the electronic memory.

Alternatively, the computer processor may execute a computer program including a set of computer-readable instructions that perform the functions described herein, the program being stored in any of the above-described non-transitory electronic memories and/or a hard disk drive, CD, DVD, FLASH drive or any other known storage media. Further, the computer-readable instructions may be provided as a utility application, background daemon, or component of an operating system, or combination thereof, executing in conjunction with a processor, such as a Xenon processor from Intel of America or an Opteron processor from AMD of America and an operating system, such as Microsoft VISTA, UNIX, Solaris, LINUX, Apple, MAC-OSX and other operating systems known to those skilled in the art.

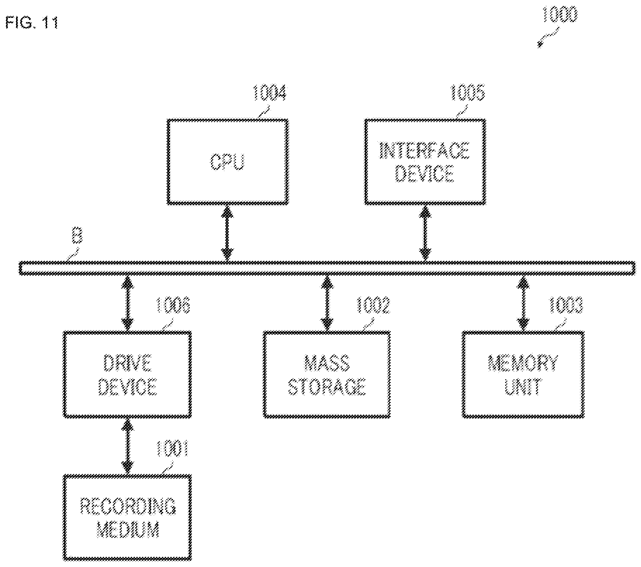

In addition, certain features of the embodiments can be implemented using a computer based system (Figure 11). The computer 1000 includes a bus B or other communication mechanism for communicating information, and a processor/CPU 1004 coupled with the bus B for processing the information. The computer 1000 also includes a main memory/memory unit 1003, such as a random access memory (RAM) or other dynamic storage device (e.g., dynamic RAM (DRAM), static RAM (SRAM), and synchronous DRAM (SDRAM)), coupled to the bus B for storing information and instructions to be executed by processor/CPU 1004. In addition, the memory unit 1003 may be used for storing temporary variables or other intermediate information during the execution of instructions by the CPU 1004. The computer 1000 may also further include a read only memory (ROM) or other static storage device (e.g., programmable ROM (PROM), erasable PROM (EPROM), and electrically erasable PROM (EEPROM)) coupled to the bus B for storing static information and instructions for the CPU 1004.

The computer 1000 may also include a disk controller coupled to the bus B to control one or more storage devices for storing information and instructions, such as mass storage 1002, and drive device 1006 (e.g., floppy disk drive, read-only compact disc drive, read/write compact disc drive, compact disc jukebox, tape drive, and removable magneto-optical drive). The storage devices may be added to the computer 1000 using an appropriate device interface (e.g., small computer system interface (SCSI), integrated device electronics (IDE), enhanced-IDE (E-IDE), direct memory access (DMA), or ultra-DMA).

The computer 1000 may also include special purpose logic devices (e.g., application specific integrated circuits (ASICs)) or configurable logic devices (e.g., simple programmable logic devices (SPLDs), complex programmable logic devices (CPLDs), and field programmable gate arrays (FPGAs)).

The computer 1000 may also include a display controller coupled to the bus B to control a display, such as a cathode ray tube (CRT), for displaying information to a computer user. The computer system includes input devices, such as a keyboard and a pointing device, for interacting with a computer user and providing information to the processor. The pointing device, for example, may be a mouse, a trackball, or a pointing stick for communicating direction information and command selections to the processor and for controlling cursor movement on the display. In addition, a printer may provide printed listings of data stored and/or generated by the computer system.

The computer 1000 performs at least a portion of the processing steps of the disclosure in response to the CPU 1004 executing one or more sequences of one or more instructions contained in a memory, such as the memory unit 1003. Such instructions may be read into the memory unit from another computer readable medium, such as the mass storage 1002 or a removable media 1001. One or more processors in a multi-processing arrangement may also be employed to execute the sequences of instructions contained in memory unit 1003. In alternative embodiments, hard-wired circuitry may be used in place of or in combination with software instructions. Thus, embodiments are not limited to any specific combination of hardware circuitry and software.

As stated above, the computer 1000 includes at least one computer readable medium 1001 or memory for holding instructions programmed according to the teachings of the disclosure and for containing data structures, tables, records, or other data described herein. Examples of computer readable media are compact discs, hard disks, floppy disks, tape, magneto-optical disks, PROMs (EPROM, EEPROM, flash EPROM), DRAM, SRAM, SDRAM, or any other magnetic medium, compact discs (e.g., CD-ROM), or any other medium from which a computer can read.

Stored on any one or on a combination of computer readable media, the present disclosure includes software for controlling the main processing unit 1004, for driving a device or devices for implementing the disclosure, and for enabling the main processing unit 1004 to interact with a human user. Such software may include, but is not limited to, device drivers, operating systems, development tools, and applications software. Such computer readable media further includes the computer program product of the present disclosure for performing all or a portion (if processing is distributed) of the processing performed in implementing the disclosure

The computer code elements on the medium of the present disclosure may be any interpretable or executable code mechanism, including but not limited to scripts, interpretable programs, dynamic link libraries (DLLs), Java classes, and complete executable programs. Moreover, parts of the processing of the present disclosure may be distributed for better performance, reliability, and/or cost.

The term "computer readable medium" as used herein refers to any medium that participates in providing instructions to the CPU 1004 for execution. A computer readable medium may take many forms, including but not limited to, non-volatile media, and volatile media. Non-volatile media includes, for example, optical, magnetic disks, and magneto-optical disks, such as the mass storage 1002 or the removable media 1001. Volatile media includes dynamic memory, such as the memory unit 1003.

Various forms of computer readable media may be involved in carrying out one or more sequences of one or more instructions to the CPU 1004 for execution. For example, the instructions may initially be carried on a magnetic disk of a remote computer. An input coupled to the bus B can receive the data and place the data on the bus B. The bus B carries the data to the memory unit 1003, from which the CPU 1004 retrieves and executes the instructions. The instructions received by the memory unit 1003 may optionally be stored on mass storage 1002 either before or after execution by the CPU 1004.

The computer 1000 also includes a communication interface 1005 coupled to the bus B. The communication interface 1004 provides a two-way data communication coupling to a network that is connected to, for example, a local area network (LAN), or to another communications network such as the Internet. For example, the communication interface 1005 may be a network interface card to attach to any packet switched LAN. As another example, the communication interface 1005 may be an asymmetrical digital subscriber line (ADSL) card, an integrated services digital network (ISDN) card or a modem to provide a data communication connection to a corresponding type of communications line. Wireless links may also be implemented. In any such implementation, the communication interface 1005 sends and receives electrical, electromagnetic or optical signals that carry digital data streams representing various types of information.

The network typically provides data communication through one or more networks to other data devices. For example, the network may provide a connection to another computer through a local network (e.g., a LAN) or through equipment operated by a service provider, which provides communication services through a communications network. The local network and the communications network use, for example, electrical, electromagnetic, or optical signals that carry digital data streams, and the associated physical layer (e.g., CAT 5 cable, coaxial cable, optical fiber, etc). Moreover, the network may provide a connection to a mobile device such as a personal digital assistant (PDA) laptop computer, or cellular telephone.

While certain embodiments have been described, these embodiments have been presented by way of example only, and are not intended to limit the scope of the invention. Indeed the systems described herein may be embodied in a variety of other forms; furthermore, various omissions, substitutions, and changes in the form of the systems described herein may be made without departing from the invention. As used herein the words "a" and "an" and the like carry the meaning of "one or more." The accompanying claims are intended to cover such forms or modifications as would fall within the scope of the invention.

## Claims

1. A system for tracking and control in medical procedures, the system comprising:
a device configured to deploy (S1001) fluorescent markers (451, 452) on at least one of an organ (111) under surgery and a surgical tool (110);
a visual light source (104);
a fluorescent light source (102) corresponding to an excitation wavelength of the fluorescent markers;
an image acquisition and control element (106) configured to:
control the visual light source and the fluorescent light source, and
capture (S1003) and digitize resulting visual images and fluorescent images; and
an image-based tracking module (107) configured to apply (S1004) image processing to the visual and fluorescent images in order to:
detect fluorescent markers on at least one of the organ and the surgical tool, and
track (S1005) coordinates of those markers in real-time.

2. The system of claim 1, further comprising:
a surgical robot (101); and
a visual servoing control module (108) configured to receive tracking information from the image-based tracking module and to control the surgical robot, based on the tracking information, to perform a surgical operation, preferably the visual servoing control module is further configured to receive manual input and to control the surgical robot, based on the manual input, to perform a surgical operation.

3. The system of claim 2, further comprising:
a manual control module configured to enable manual control of the surgical robot in place of control by the visual servoing control module.

4. The system of claim 1, further comprising:
a surgical robot (101); and
a manual control module configured to receive manual input and execute master-slave control of the surgical robot.

5. The system of claim 1, further comprising:
a display (109) configured to display (S1006) at least one of the visual images and the fluorescent images, preferably wherein the display is stereoscopic or monoscopic.

6. The system of claim 1, wherein the image-based tracking module further identifies the organ or the surgical tool based on the detected fluorescent markers.

7. The system of claim 1, wherein the image acquisition and control element further comprises:
a dynamic tunable filter (103) configured to alternatively pass visual light and light emitted by the fluorescent markers, and
a charged coupled device (105) configured to capture at least one of visual images and fluorescent images.

8. The system of claim 1, wherein the image acquisition and control element generates stereoscopic or monoscopic images.

9. The system of claim 5, wherein the display is stereoscopic, is further configured to display (S1006) visual images and a color coded overlay of fluorescent images, and is preferably further configured to display (S1006) an augmented reality image by overlaying target points detected by the image-based tracking module.

10. The system of claim 1, wherein the system is configured to provide (S1007) at least one of visual, audio, and haptic feedback to a system operator, based on information provided by the image-based tracking module.

11. The system of claim 1, wherein the system is configured to operate in each of a manual mode, a semi-autonomous mode, and an autonomous mode.

12. The system of claim 1, wherein the image-based tracking module identifies virtual boundaries based on the detected fluorescent markers to designate critical structures.

13. The system of claim 12, further comprising:
a detection device configured to determine whether a surgical tool has passed a boundary and to provide constraints on motion or provide alarms when the boundary has been crossed in order to protect the critical structures.

14. The system of claim 1, wherein the fluorescent light source is a near-infrared (NIR) light source.

15. The system of claim 1, wherein the device that deploys the fluorescent markers is configured to deploy the fluorescent markers by spraying, painting, attachment, tissue injection, or intravenous injection.

## Patentansprüche

1. System zum Verfolgen und Steuern in medizinischen Eingriffen, wobei das System Folgendes umfasst:
eine Vorrichtung, die konfiguriert ist, Fluoreszenzmarker (451, 452) auf einem Organ (111) während einer Operation und/oder auf einem chirurgischen Instrument (110) einzusetzen (S1001);
eine sichtbare Lichtquelle (104);
eine fluoreszierende Lichtquelle (102), die einer Anregungswellenlänge der Fluoreszenzmarker entspricht;
ein Bildaufnahme- und Steuerelement (106), das konfiguriert ist zum:
Steuern der sichtbaren Lichtquelle und der fluoreszierenden Lichtquelle und
Erfassen (S1003) und Digitalisieren resultierender visueller Bilder und fluoreszierender Bilder; und
ein bildbasiertes Verfolgungsmodul (107), das konfiguriert ist, Bildverarbeitung auf die visuellen und fluoreszierenden Bilder anzuwenden (S1004), zum:
Erfassen von Fluoreszenzmarkern auf dem Organ und/oder dem chirurgischen Instrument und
Verfolgen (S1005) von Koordinaten dieser Marker in Echtzeit.

2. System nach Anspruch 1, ferner Folgendes umfassend:
einen Operationsroboter (101); und
ein visuelles Servosteuerungsmodul (108), das konfiguriert ist, Verfolgungsinformationen von dem bildbasierten Verfolgungsmodul zu erhalten und den Operationsroboter auf der Grundlage der Verfolgungsinformationen zu steuern, um einen chirurgischen Eingriff durchzuführen, vorzugsweise wobei das visuelle Servosteuerungsmodul ferner konfiguriert ist, eine manuelle Eingabe zu erhalten und den Operationsroboter auf der Grundlage der manuellen Eingabe zu steuern, um einen chirurgischen Eingriff durchzuführen.

3. System nach Anspruch 2, ferner Folgendes umfassend:
ein manuelles Steuermodul, das konfiguriert ist, ein manuelles Steuern des Operationsroboters anstelle von Steuern durch das visuelle Servosteuerungsmodul zu ermöglichen.

4. System nach Anspruch 1, ferner Folgendes umfassend:
einen Operationsroboter (101); und
ein manuelles Steuerungsmodul, das konfiguriert ist, eine manuelle Eingabe zu erhalten und eine Master-Slave-Ansteuerung des Operationsroboters auszuführen.

5. System nach Anspruch 1, ferner Folgendes umfassend:
eine Anzeige (109), die konfiguriert ist, die visuellen Bilder und/oder die fluoreszierenden Bilder anzuzeigen (S1006), vorzugsweise wobei die Anzeige stereoskopisch oder monoskopisch ist.

6. System nach Anspruch 1, wobei das bildbasierte Verfolgungsmodul ferner das Organ oder das chirurgische Instrument auf der Grundlage der erfassten Fluoreszenzmarker identifiziert.

7. System nach Anspruch 1, wobei das Bildaufnahme- und Steuerelement ferner Folgendes umfasst:
einen dynamischen abstimmbaren Filter (103), der konfiguriert ist, sichtbares Licht und durch die Fluoreszenzmarker abgegebenes Licht abwechselnd durchzulassen, und
ein ladungsgekoppeltes Bauelement (105), das konfiguriert ist, visuelle Bilder und/oder fluoreszierende Bilder aufzunehmen.

8. System nach Anspruch 1, wobei das Bildaufnahme- und Steuerelement stereoskopische oder monoskopische Bilder erzeugt.

9. System nach Anspruch 5, wobei die Anzeige stereoskopisch ist, ferner konfiguriert ist, visuelle Bilder und ein farbkodiertes Overlay von fluoreszierenden Bildern anzuzeigen (S1006) und vorzugsweise ferner konfiguriert ist, ein Bild erweiterter Realität durch Überlagern von durch das bildbasierte Verfolgungsmodul erfassten Zielpunkten anzuzeigen (S1006).

10. System nach Anspruch 1, wobei das System konfiguriert ist, visuelles, akustisches und/oder haptisches Feedback auf der Grundlage von durch das bildbasierte Verfolgungsmodul bereitgestellten Informationen an einen Systembetreiber bereitzustellen (S1007).

11. System nach Anspruch 1, wobei das System konfiguriert ist, jeweils in einem manuellen Modus, einem halbautonomen Modus und einem autonomen Modus betrieben zu werden.

12. System nach Anspruch 1, wobei das bildbasierte Verfolgungsmodul virtuelle Grenzen auf der Grundlage der erfassten Fluoreszenzmarker identifiziert, um wesentliche Strukturen zu markieren.

13. System nach Anspruch 12, ferner Folgendes umfassend:
eine Erfassungsvorrichtung, die konfiguriert ist, zu bestimmen, ob ein chirurgisches Instrument eine Grenze überschritten hat, und Beschränkungen in der Bewegung bereitzustellen oder Warnmeldungen bereitzustellen, wenn die Grenze überquert worden ist, um die wesentlichen Strukturen zu schützen.

14. System nach Anspruch 1, wobei die fluoreszierende Lichtquelle eine Nah-Infrarot(NIR)-Lichtquelle ist.

15. System nach Anspruch 1, wobei die Vorrichtung, die die Fluoreszenzmarker einsetzt, konfiguriert ist, die Fluoreszenzmarker durch Sprühen, Streichen, Befestigen, Einspritzen in Gewebe oder intravenöses Einspritzen einzusetzen.

## Revendications

1. Système de suivi et de commande dans des procédures médicales, le système comprenant :
un dispositif configuré pour déployer (S1001) des marqueurs fluorescents (451, 452) sur au moins l'un d'un organe (111) en cours d'opération et d'un outil chirurgical (110) ;
une source de lumière visuelle (104) ;
une source de lumière fluorescente (102) correspondant à une longueur d'onde d'excitation des marqueurs fluorescents ;
un élément d'acquisition et de commande d'image (106) configuré pour :
commander la source de lumière visuelle et la source de lumière fluorescente, et
capturer (S1003) et numériser des images visuelles et des images fluorescentes résultantes ; et
un module de suivi par image (107) configuré pour appliquer (S1004) un traitement d'image aux images visuelles et fluorescentes afin de :
détecter des marqueurs fluorescents sur au moins l'un de l'organe et de l'outil chirurgical, et
suivre (S1005) des coordonnées de ces marqueurs en temps réel.

2. Système selon la revendication 1, comprenant en outre :
un robot chirurgical (101) ; et
un module de commande d'asservissement visuel (108) configuré pour recevoir des informations de suivi en provenance du module de suivi par image et pour commander le robot chirurgical, d'après les informations de suivi, pour réaliser une opération chirurgicale, de préférence le module de commande d'asservissement visuel est en outre configuré pour recevoir une entrée manuelle et pour commander le robot chirurgical, d'après l'entrée manuelle, pour réaliser une opération chirurgicale.

3. Système selon la revendication 2, comprenant en outre :
un module de commande manuelle configuré pour permettre une commande manuelle du robot chirurgical au lieu d'une commande par le module de commande d'asservissement visuel.

4. Système selon la revendication 1, comprenant en outre :
un robot chirurgical (101) ; et
un module de commande manuelle configuré pour recevoir une entrée manuelle et exécuter une commande maître-esclave du robot chirurgical.

5. Système selon la revendication 1, comprenant en outre :
un afficheur (109) configuré pour afficher (S1006) au moins l'une des images visuelles et des images fluorescentes, de préférence dans lequel l'afficheur est stéréoscopique ou monoscopique.

6. Système selon la revendication 1, dans lequel le module de suivi par image identifie en outre l'organe ou l'outil chirurgical d'après les marqueurs fluorescents détectés.

7. Système selon la revendication 1, dans lequel l'élément d'acquisition et de commande d'image comprend en outre :
un filtre accordable dynamique (103) configuré pour faire passer alternativement la lumière visuelle et la lumière émise par les marqueurs fluorescents, et
un dispositif à transfert de charge (105) configuré pour capturer au moins l'une des images visuelles et des images fluorescentes.

8. Système selon la revendication 1, dans lequel l'élément d'acquisition et de commande d'image génère des images stéréoscopiques ou monoscopiques.

9. Système selon la revendication 5, dans lequel l'afficheur est stéréoscopique, est en outre configuré pour afficher (S1006) des images visuelles et une superposition à code couleur d'images fluorescentes, et est en outre de préférence configuré pour afficher (S1006) une image à réalité augmentée en superposant des points cibles détectés par le module de suivi par image.

10. Système selon la revendication 1, dans lequel le système est configuré pour fournir (S1007) au moins l'une d'une rétroaction visuelle, audio et haptique à un opérateur système, d'après des informations fournies par le module de suivi par image.

11. Système selon la revendication 1, dans lequel le système est configuré pour fonctionner dans chacun d'un mode manuel, d'un mode semi-autonome et d'un mode autonome.

12. Système selon la revendication 1, dans lequel le module de suivi par image identifie des limites virtuelles d'après les marqueurs fluorescents détectés pour désigner des structures critiques.

13. Système selon la revendication 12, comprenant en outre :
un dispositif de détection configuré pour déterminer si un outil chirurgical a dépassé une limite et pour fournir des contraintes de mouvement ou fournir des alarmes lorsque la limite a été traversée afin de protéger les structures critiques.

14. Système selon la revendication 1, dans lequel la source de lumière fluorescente est une source de lumière de proche infrarouge (NIR).

15. Système selon la revendication 1, dans lequel le dispositif qui déploie les marqueurs fluorescents est configuré pour déployer les marqueurs fluorescents par pulvérisation, peinture, fixation, injection dans le tissu ou injection intraveineuse.
